# EUROPÄISCHE PATENTSCHRIFT

(11) **EP 4 041 093 B1**
(45) Veröffentlichungstag und Bekanntmachung des Hinweises auf die Patenterteilung: **09.08.2023**
(21) Anmeldenummer: 20793278.1
(22) Anmeldetag: 08.10.2020
(51) Int. Cl.: A61B 10/06, A61B 10/02, A61B 17/28

(54) **CHIRURGISCHES INSTRUMENT, VERFAHREN ZUR HERSTELLUNG EINES CHIRURGISCHEN INSTRUMENTS UND VERWENDUNG EINES DREHGELENKS ZUR AUSBILDUNG EINES SCHNEIDEWERKZEUGS EINES CHIRURGISCHEN INSTRUMENTS**
SURGICAL INSTRUMENT, METHOD FOR PRODUCING A SURGICAL INSTRUMENT, AND USE OF A ROTARY JOINT FOR FORMING A CUTTING TOOL OF A SURGICAL INSTRUMENT
INSTRUMENT CHIRURGICAL, PROCÉDÉ DE PRODUCTION D'UN INSTRUMENT CHIRURGICAL ET UTILISATION D'UNE ARTICULATION ROTOÏDE POUR FORMER UN OUTIL DE COUPE D'UN INSTRUMENT CHIRURGICAL

(30) Priorität: 08.10.2019 DE 102019126965
(43) Veröffentlichungstag der Anmeldung: 17.08.2022
(73) Patentinhaber: Morpheus AG, 78532 Tuttlingen (DE)
(72) Erfinder: RACK, Timo, 78604 Rietheim-Weilheim (DE); BLESSING, Heiko, 78591 Durchhausen (DE)
(74) Vertreter: Mertzlufft-Paufler, Cornelius
(86) Internationale Anmeldenummer: PCT/EP2020/078286
(87) Internationale Veröffentlichungsnummer: WO 2021/069594

(56) Entgegenhaltungen:
- EP-A1- 1 161 183
- DE-A1-102015 112 716
- DE-A1-102015 114 306
- DE-A1-102018 102 854
- US-A1- 2004 068 291
- US-A1- 2005 267 503

## Beschreibung

Die Erfindung betrifft ein chirurgisches Instrument zum Abtrennen und Entfernen eines Bioptats (auch als Gewebeprobe bezeichnet), wobei in einem Schaft ein Absaugkanal zum Absaugen des Bioptats aus einem distalen Bereich in Richtung eines proximalen Bereichs ausgebildet ist.

Chirurgische Instrument dieser Art sind bereits bekannt und können zum Beispiel als Gewebestanzen ausbildet sein. Diese Art der chirurgischen Instrumente weisen eine Doppelfunktion auf. Sie dienen einerseits zur Abtrennung eines Gewebestücks. Des Weiteren soll durch die chirurgischen Instrumente jedoch nicht nur die Abtrennung möglich sein, sondern direkt ein Entfernen der Gewebeprobe vom Ort der Abtrennung in einen zum Auffangen der Gewebeprobe vorgesehenen Kollektor. Hierzu weisen die chirurgischen Instrumente den zuvor genannten Absaugkanal auf, der mit dem Schneidewerkzeug gekoppelt ist, um die abgetrennte Gewebeprobe direkt vom Ort der Abtrennung absaugen zu können, ohne dass es dabei erforderlich ist, dass der Chirurg oder zusätzliches Personal die Gewebeprobe per Hand entnehmen muss.

Die Begriffe "distal" und "proximal" können jeweils in Bezug auf eine vorgesehene Nutzungssituation des chirurgischen Instruments zu verstehen sein. Der Begriff "distal" kann hierbei dahingehend verstanden werden, dass damit eine von einer Hand eines Benutzers, mit welcher dieser das chirurgische Instrument hält, entfernte Lage gemeint ist und/oder der Begriff "proximal" kann hierbei dahingehend verstanden werden, dass damit eine von einer Hand eines Benutzers, mit welcher dieser das chirurgische Instrument hält, nahe Lage gemeint ist.

Es hat sich jedoch gezeigt, dass die Absaugung des Bioptats in der Praxis häufig fehlschlägt. Regelmäßig kommt es daher bei der Verwendung chirurgischer Instrumente eingangs genannter Art vor, dass mittels des chirurgischen Instruments abgetrennte Gewebeproben beim Absaugen stecken bleiben und der Chirurg dann für den Zeitraum der Behebung der Verstopfung oder dem Austausch des chirurgischen Instrumentes die Operation unterbrechen muss. Im schlimmsten Fall kann es vorkommen, dass das entnommene Bioptat anschließend nicht mehr zur weiteren Diagnostik verwendbar ist, da es z.B. beim Entfernen der Verstopfung unbrauchbar wird.

Die Erfindung betrifft zudem ein chirurgisches Instrument zum Abtrennen und Entfernen eines Bioptats, wobei in einem distalen Bereich des Schaftes ein Schneidewerkzeug ausgebildet ist, das ein feststehendes Schneidteil und ein bewegliches Gegenschneidteil, insbesondere ein um eine durch ein Drehgelenk ausgebildete Drehachse, relativ zum feststehenden Schneidteil verstellbares Gegenschneidteil, aufweist.

Des Weiteren betrifft die Erfindung ein Verfahren zur Herstellung eines solchen chirurgischen Instruments und die Verwendung eines Drehgelenks zur Ausbildung eines Schneidewerkzeugs eines solchen chirurgischen Instruments.

Aus DE 10 2015 112716 A1, US 2005 264 503 A1, DE 10 2018 102854 A1, US 2004 / 068291 A1 und DE 10 2015 114306 A1 sind wie eingangs eingeführte Instrumente mit unterschiedlichsten Schneidwerkzeugen zum Abtrennen von Bioptaten vorbekannt. Werden Drehgelenke für das jeweilige Schneidwerkzeug verwendet, so werden die Befestigungselemente, die die Drehachse des Drehgelenks definieren, typischerweise dadurch befestigt, dass eine Materialverdickung an dem Befestigungselement ausgebildet wird, die hervorsteht und so eine Verankerung des Befestigungselement in einem jeweiligen notwendigen Durchgangsloch bewirkt. Beispielsweise schlägt WO 00/54658 A1 zur Ausbildung eines Drehgelenks vor, zwei Befestigungselemente von außen nach innen in eine Wandung eines Absaugkanals einzuführen, Wobei die Befestigungselemente durch ein jeweilige Materialverdickung in Position gehalten werden. EP 1 161 183 A1 ist der nächstliegender Stand der Technik und offenbart die Merkmale des Oberbegriffs von Anspruch 1.

Von diesem technischen Hintergrund ausgehend liegt der Erfindung die Aufgabe zu Grunde, ein chirurgisches Instrument eingangs genannter Art mit verbesserten Gebrauchseigenschaften zu schaffen.

Die Lösung dieser Aufgabe wird erfindungsgemäß durch ein chirurgisches Instrument eingangs genannter Art mit den Merkmalen nach Anspruch 1 gelöst.

Bei einem chirurgischen Instrument eingangs genannter Art kann beispielsweise vorgesehen sein, dass sich ein Durchmesser des Querschnitts des Absaugkanals in Absaugrichtung vom distalen Bereich zum proximalen Bereich erweitert. Ein abgetrenntes Bioptat kann somit durch Ansaugen beispielsweise durch Verwendung einer geeigneten Pumpe und/oder durch Anschließen des Absaugkanals über ein Anschlussteil an eine externe Pumpe vom distalen Bereich des Schaftes durch den Absaugkanal zum proximalen Bereich beispielsweise bis zu einer Austrittsöffnung transportiert werden. Über die Austrittsöffnung kann die Gewebeprobe schließlich in einen Gewebekollektor überführt werden. Aufgrund der Querschnittserweiterung des Absaugkanals kann ein Verstopfen des Absaugkanals besser verhindert werden, da das Bioptat nach dem Aufbau einer ausreichenden Saugkraft am distalen Bereich, insbesondere am Schneidwerkzeug, in den Absaugkanal hineingesaugt wird, eine durch Entlangrutschen an einer Wandung des Absaugkanals entstehende Reibung aufgrund der Querschnittserweiterung des Absaugkanals jedoch minimiert werden kann. Das Bioptat wirkt dabei selbst wie eine Art Stopfen, der sich nach Überschreiten eines Schwellenwertes der Ansaugkraft durch Aufbau eines Unterdrucks löst und abgesaugt wird. Ein Innendurchmesser des Schneidwerkzeuges kann somit dem Durchmesser des Querschnitts des Absaugkanals am distalen Ende entsprechen.

Der Begriff "Querschnitt des Absaugkanals" kann sich dabei auf eine vorzugsweise sich senkrecht zu einer Längsachse des Absaugkanals erstreckende Ausdehnung eines Hohlraums zwischen zwei sich gegenüberliegenden Wandungen des Absaugkanals beziehen.

Zur Lösung der Aufgabe wird erfindungsgemäß ein chirurgisches Instrument eingangs genannter Art vorgeschlagen, wobei ein (beispielsweise das bereits zuvor genannte) Drehgelenk durch zwei Befestigungselemente ausgebildet ist, wobei die Befestigungselemente jeweils von innen nach außen eingeführt sind. Ferner ist vorgesehen, dass die Stirnseiten der Befestigungselemente zusammen mit einer Wandung des Absaugkanals die planare Absaugkanalsinnenseite bilden.

Hierbei bezieht sich innen auf eine Absaugkanalsinnenseite und/oder außen auf eine Außenseite des Schaftes. Somit kann verhindert werden, dass die Befestigungselemente in den Absaugkanal zu weit hineinragen und/oder das Bioptat beim Absaugen an den Befestigungselementen hängen bleibt.

Bei vorbekannten chirurgischen Instrumenten ragen die Befestigungselemente oftmals wenigstens teilweise in den Absaugkanal hinein oder führen durch diesen sogar hindurch. Dadurch stellen diese ein Hindernis dar, das zu Verstopfung des Absaugkanals durch eine Gewebeprobe führen kann. Durch die erfindungsgemäße Lösung kann die Gewebeprobe ungehindert abgesaugt werden.

Vorzugsweise können die Stirnseiten der Köpfe der Befestigungselemente zusammen mit einer Wandung des Absaugkanals die planare Absaugkanalsinnenseite bilden. Insbesondere kann dabei ein Übergang zwischen einem Kopf und der Wandung glatt und/oder stufenlos ausgestaltet sein. Somit ist es nicht mehr möglich, dass Verstopfungen des Absaugkanals durch sich festgesetzte, mittels des Schneidwerkzeuges abgetrennte Gewebeproben auftreten. Ferner kann besser verhindert werden, dass es zu einer Beeinträchtigung des Bioptats durch eine Irritation an einer scharfkantigen Stelle des im Absaugkanal liegenden Teils des Befestigungselements kommt.

Der Begriff "Kopf des Befestigungselements" kann sich auf einen Teil des Befestigungselements beziehen, der an einem gegenüberliegenden Ende zu dem in eine Öffnung eingeführten Teil (Einführungsteil) des Befestigungselements angeordnet oder ausgebildet ist. Vorzugsweise kann der Kopf des Befestigungselements wenigstens teilweise einen breiteren Durchmesser als der Einführungsteil aufweisen. Das Befestigungselement kann zum Beispiel als Niet, Bolzen, Schraube und/oder Nagel ausgebildet sein.

Es kann vorgesehen sein, dass eine Bohrung für eines der Befestigungselemente oder Bohrungen für die zwei Befestigungselemente in einer Wandung des Absaugkanals, insbesondere in der bereits zuvor erwähnte Wandung, innenseitig einen größeren Durchmesser hat oder haben als außenseitig. Hierbei kann ein Übergang der Bohrung von einer Außenseite der Wandung zu einer Innenseite der Wandung durch eine Stufe erfolgen. Die Stufe bildet einen Anschlag für das von innen nach außen eingesetzte Befestigungsmittel. Alternativ kann auch der Übergang konisch erfolgen. Auch ein stetiger Übergang ist möglich, indem beispielsweise der Übergang Rundungen aufweist. Das Befestigungsmittel und die Bohrung sind dabei bevorzugt aufeinander abgestimmt.

Nachfolgend werden vorteilhafte Ausgestaltungen der Erfindung beschrieben, die allein oder in Kombination mit den Merkmalen anderer Ausgestaltungen zusammen mit den Merkmalen nach Anspruch 1 kombiniert werden können.

Gemäß einer vorteilhaften Ausgestaltung kann es vorgesehen sein, dass sich der Querschnitt des Absaugkanals in einem Erweiterungsbereich erweitert. Insbesondere kann sich der Querschnitt des Absaugkanals in einem Erweiterungsbereich kontinuierlich und/oder geradlinig erweitern. Somit kann sich nach dem Abtrennen der Gewebeprobe zunächst eine ausreichend große Ansaugkraft innerhalb des, insbesondere anfangs durch die Gewebeprobe verstopften, Absaugkanals aufgebaut werden, bevor die Gewebeprobe bei Erreichen eines Schwellenwertes, in welchem die Ansaugkraft größer als die Reibungskraft ist, die Gewebeprobe durch den Absaugkanals gesaugt wird. Der Erweiterungsbereich kann entlang der Längsrichtung des Absaugkanals beabstandet vom Schneidwerkzeug und/oder unmittelbar ans Schneidwerkzeug angrenzend ausgebildet sein. Somit kann auf einfache Weise eine unterschiedliche Reibungskraft zwischen der Gewebeprobe und der Wandung des Absaugkanals definiert werden.

Gemäß einer weiteren vorteilhaften Ausgestaltung kann es vorgesehen sein, dass der Absaugkanal vor und/oder nach einem Erweiterungsbereich, beispielsweise dem bereits zuvor genannten Erweiterungsbereich, einen gleichdimensionierten Querschnitt und/oder in der Form gleichbleibenden Querschnitt aufweist. Insbesondere kann der Absaugkanal vor und/oder nach dem Erweiterungsbereich zylindrisch ausgebildet sein. Somit gleitet das Bioptat besonders reibungsarm durch den Absaugkanal. Gegenüber einem Absaugkanal mit gleichbleibenden Querschnittsmaßen weist der Absaugkanal des chirurgischen Instruments den Vorteil auf, dass es durch diese noch immer möglich ist, zunächst eine Ansaugkraft durch anfängliches Verstopfen des Absaugkanals mittels der Gewebeprobe aufzubauen, dass jedoch nach Erreichen einer ausreichend hohen Ansaugkraft und Abtransport der Gewebeprobe die Reibung zwischen der Gewebeprobe und einer Wandung des Absaugkanals über die restliche Transportstrecke möglichst gering gehalten werden kann.

Gemäß einer weiteren vorteilhaften Ausgestaltung kann es vorgesehen sein, dass sich ein Erweiterungsbereich, beispielsweise der bereits zuvor genannte Erweiterungsbereich, von einem distalen Ende des Schaftes bis maximal zu einer Mitte des Schaftes, insbesondere bis maximal zu einem Drittel einer Länge des Schaftes, erstreckt. Somit kann der Erweiterungsbereich genutzt werden, um eine anfängliche, gewollte Verstopfung herbeizuführen, um eine Ansaugkraft aufzubauen, während sich das Bioptat innerhalb des Erweiterungsbereichs befindet. Bei vorbekannten chirurgischen Instrumenten mit einem Absaugkanal, der einen zumindest nahezu durchgehend gleichbleibend breiten Querschnitt aufweist, wird ebenfalls eine anfängliche Verstopfung hervorgerufen. Allerdings kann es hierbei vorkommen, dass die Gewebeprobe bei einem Weitertransport innerhalb des Absaugkanal stecken bleibt, wobei sich dann in diesem Zustand mitunter ein Verschluss des Absaugkanal durch das Bioptat einstellt und das Bioptat somit im Absaugkanal festsitzt.

Gemäß einer weiteren vorteilhaften Ausgestaltung kann es vorgesehen sein, dass die Stirnseiten der Befestigungselemente in einer Absaugkanalsinnenseite, beispielsweise der bereits zuvor genannten Absaugkanalsinnenseite, liegen. Insbesondere können die Köpfe der Befestigungselemente in der Absaugkanalsinnenseite liegen. Somit ist eine einfache konstruktive Lösung geschaffen, durch welche die oben genannte Aufgabe gelöst wird und welche zur Narbenproduktion des chirurgischen Instruments geeignet ist.

Gemäß einer weiteren vorteilhaften Ausgestaltung kann es vorgesehen sein, dass der Schaft eine Führungsschiene und eine relativ zur Führungsschiene verstellbare Gleitschiene aufweist. Alternativ oder ergänzend dazu kann es gemäß einer weiteren vorteilhaften Ausgestaltung vorgesehen sein, dass der Absaugkanal durch eine Führungsschiene, beispielsweise die bereits zuvor genannte Führungsschiene, und/oder eine Gleitschiene, beispielsweise die bereits zuvor genannte Gleitschiene, ausgebildet ist. Insbesondere kann der Absaugkanal teilweise oder vollständig durch die Führungsschiene und/oder die Gleitschiene ausgebildet sein.

Um besser verhindern zu können, dass es zu einer Irritation oder Beschädigung des zu entnehmenden Bioptats beim Abtransport durch den Absaugkanal kommt, können die Kanten der Köpfe der Befestigungselemente wenigstens teilweise abgerundet und/oder die Stirnflächen konvex gewölbt sein.

Gemäß einer weiteren vorteilhaften Ausgestaltung kann es vorgesehen sein, dass der Absaugkanal einen wenigstens halbrunden, insbesondere bogenförmigen, Querschnitt aufweist.

Die Erfindung betrifft zudem ein Verfahren zur Herstellung eines chirurgischen Instruments, wie es hierin beschrieben und beansprucht ist, mit einem Schneidewerkzeug, das ein Drehgelenk aufweist. Dabei ist es erfindungsgemäß vorgesehen, dass ein zur Ausbildung des Drehgelenks verwendetes Befestigungselement durch eine Wandung eines Absaugkanals von innen, also eine Absaugkanalinnenseite, nach außen in eine Öffnung eingeführt und darin fixiert wird. Dabei bilden eine Stirnseite des Befestigungselements und eine Innenseite der Wandung eine planare Absaugkanalsinnenseite aus.

Das erfindungsgemäße Verfahren weist somit den Vorteil auf, dass dadurch eine wenige Arbeitsschritte und einen geringen Materialaufwand erfordernde Fertigung eines chirurgischen Instruments möglich ist. Somit kann der Kostenaufwand für die Herstellung gegenüber vorbekannten Herstellungsverfahren deutlich reduziert werden, da insbesondere aufwändige Nachbearbeitungsschritte am Absaugkanal ausbleiben können. Beispielsweise kann dabei auf ein Abschleifen eines in den Absaugkanal ragenden Teils eines Befestigungselements verzichtet werden. Die Köpfe der Befestigungselemente können in eine Materialaussparung an der Absaugkanalinnenseite eingesetzt werden, um vorzugsweise eine planare Fläche mit der Wandung zu bilden.

Zur Fixierung der in die Öffnung eingeführten Befestigungselemente können diese von außen, insbesondere also an einer Außenseite des Schaftes und/oder des Schneidteils und/oder des Gegenschneidteils verschweißt werden. Optional können die Schweißpunkte und/oder die an der Außenseite überstehenden Abschnitte der Befestigungselemente anschließend abgeschliffen werden, um eine glatte Außenoberfläche zu schaffen, damit ein Operateur nicht versehentlich durch ungeschliffene, scharfkantige Bereiche Verletzungen des Patienten herbeiführt.

Die Erfindung betrifft weiter eine Verwendung eines Drehgelenks, welches zwei von innen eingesteckte Befestigungselemente aufweist, zur Ausbildung eines Schneidewerkzeugs eines chirurgischen Instruments mit einem Absaugkanal, wie es hierin beschrieben und beansprucht ist. Auch bei dieser Verwendung ist vorgesehen, dass die Befestigungselemente dazu verwendet werden, eine Absaugkanalsinnenseite des Absaugkanals planar auszugestalten. Die Erfindung wird nun anhand mehrerer Ausführungsbeispiele näher beschrieben, ist jedoch nicht auf diese Ausführungsbeispiele beschränkt. Der Schutzumfang wird ausschließlich durch die Ansprüche definiert.

Es zeigt:
- Fig. 1: eine erste mögliche Ausführungsvariante eines chirurgischen Instruments zum Abtrennen und Entfernen eines Bioptats mit einem Schneidewerkzeug, wobei sich ein Querschnitt eines Absaugkanals in Absaugrichtung vom distalen Bereich zum proximalen Bereich in einem Erweiterungsbereich kontinuierlich erweitert,
- Fig. 2: eine Draufsicht auf einen horizontalen Längsschnitt der Ausführungsvariante aus Fig. 1,
- Fig. 3: eine Seitenansicht eines vertikalen Längsschnitts der Ausführungsvariante aus Fig. 1,
- Fig. 4: eine weitere mögliche Ausführungsvariante eines chirurgischen Instruments zum Abtrennen und Entfernen eines Bioptats, wobei diese ebenfalls einen Absaugkanal mit sich wenigstens teilweise im Durchmesser in Absaugrichtung vergrößerndem Querschnitt aufweist, wobei das Schneidewerkzeug ein Drehgelenk aufweist, das zwei Befestigungselemente aufweist, wobei die Befestigungselemente jeweils von innen nach außen eingeführt sind, so dass keine scharfkantigen Teile der Befestigungselemente in den Absaugkanal ragen,
- Fig. 5: eine Frontansicht des Schaftes eines chirurgischen Instruments gemäß Fig. 4, wobei der Schaft ohne ein Griffteil zum Betätigen des Schneidewerkzeugs abgebildet ist,
- Fig. 6: eine Rückansicht des Schaftes eines chirurgischen Instruments gemäß Fig. 4, wobei der Schaft ohne eine Griffteil zum Betätigen des Schneidewerkzeugs abgebildet ist,
- Fig. 7: ein alternativ ausgestalteten distalen Bereich eines weiteren Ausführungsbeispiels eines erfindungsgemäß ausgebildeten chirurgischen Instruments mit drehbarem Schneidwerkzeug,
- Fig. 8: das in Fig. 7 gezeigte chirurgische Instrument 1 mit teilweise ausgeblendeten Komponenten.

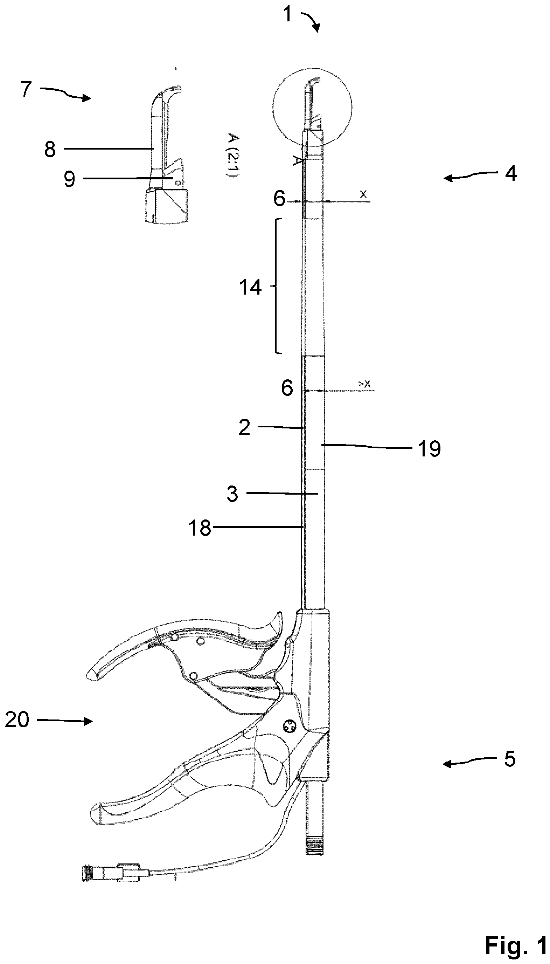

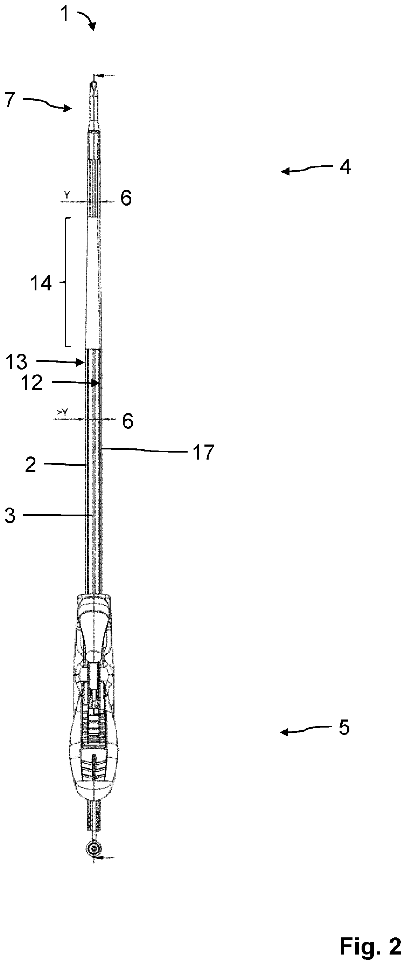

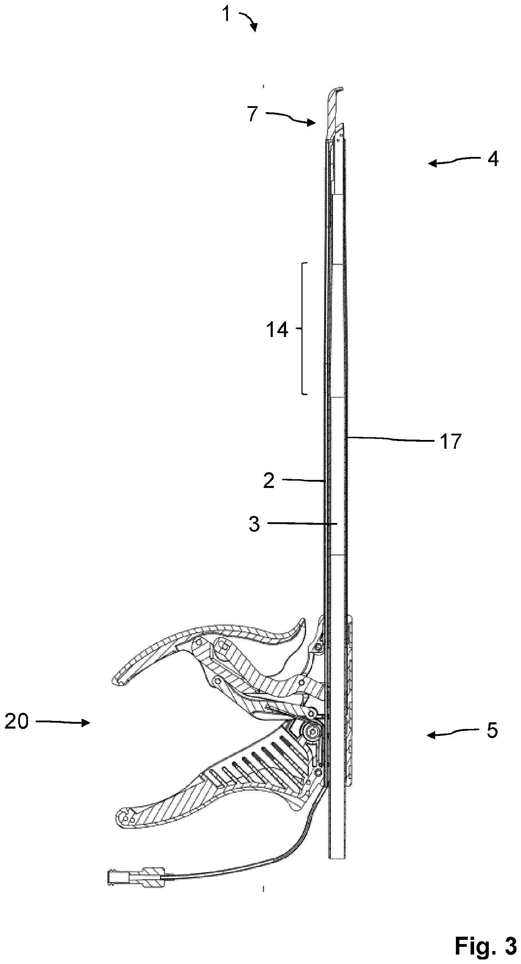

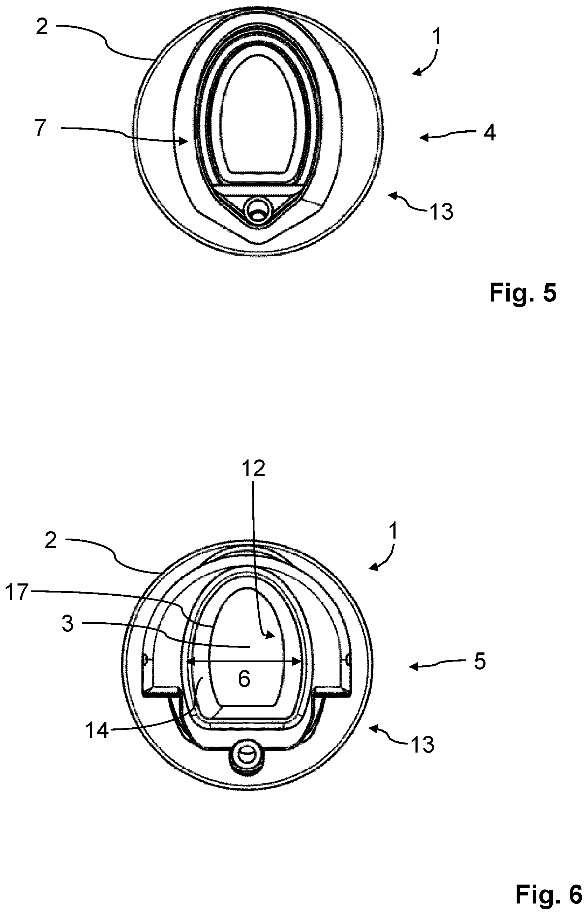

In den Figuren 1-6 sind mögliche Ausgestaltungen eines chirurgischen Instruments zum Abtrennen und Entfernen eines Bioptats gezeigt, also insbesondere von Körpermaterial, das während eines Biopsie-Eingriffs entnommen wird, wobei das chirurgische Instrument im Ganzen als 1 bezeichnet ist.

Das chirurgische Instrument 1 kann beispielsweise, wie zu Teil in den Figuren gezeigt ist, als eine Gewebestanze ausgestaltet sein.

Derartige chirurgische Instrumente 1 weisen in der Regel ein Griffteil 20 auf, durch welches ein Benutzer ein Schneidewerkzeug 8 bedienen kann, also insbesondere eine Verstellung des Schneidewerkzeugs 8 erreichen kann. Als Schneidewerkzeug 8 kann grundsätzlich jedes Werkzeug eines chirurgischen Instruments 1 verstanden werden, das zum Abtrennen von Gewebe geeignet und/oder ausgelegt ist.

Das Griffteil 20 ist dabei fest mit einem Schaft 2 verbunden oder verbindbar. Der Schaft 2 ist häufig mehrteilig aufgebaut.

Durch den Schaft 2 ist zudem ein Absaugkanal 3 ausgebildet, der dazu dient, die durch das Schneidewerkzeug 8 abgetrennten Bioptate von einem distalen Bereich 4 in Richtung eines proximalen Bereichs 5 des chirurgischen Instruments 1 transportieren zu können, insbesondere von dort absaugen zu können. Der Absaugvorgang kann dabei zum Beispiel durch Anlegen eines Unterdrucks an den Absaugkanal 4 erfolgen. Insbesondere kann das chirurgische Instrument 1 einen Anschluss zum Verbinden mit einer externen Unterdruckeinrichtung und/oder eine eigene Unterdruckeinrichtung zur Erzeugung eines Unterdrucks aufweisen.

Am proximalen Bereich 5 des Schafts 2, insbesondere am proximalen Ende des Schaftes 2, kann ein Gewebekollektor angeordnet oder anordenbar sein, um das abgesaugte Bioptat aufzufangen.

Beim Absaugen des Bioptats ist darauf zu achten, dass es nicht zu einer Zerstörung des Bioptats kommt, da das Bioptat in der Regel anschließend untersucht werden soll. Somit sollte ein zum Absaugen des Bioptats angelegter Unterdruck ein möglichst schonendes Absaugen ermöglichen, zum Beispiel indem eine Druckdifferenz zwischen dem atmosphärischen Druck und dem angelegten Unterdruck nicht zu hoch ausfällt.

Allerdings kann es vorkommen, dass bei einer zu geringen Druckdifferenz zwischen dem herrschenden Druck und dem angelegten Unterdruck nicht gewährleistet ist, dass das Bioptat abgesaugt wird, sondern es zu einer Verstopfung des Absaugkanals 3 kommt.

Dieses Problem wird dadurch gelöst, dass sich ein Querschnitt 6 des Absaugkanals 3 in Absaugrichtung vom distalen Bereich 4 zum proximalen Bereich 5 wenigstens innerhalb eines Erweiterungsbereichs 14 erweitert. Der Erweiterungsbereich 14 des Absaugkanals 3 bezieht sich somit auf den Teil, in welchem eine Veränderung des Durchmessers des Absaugkanals 3 vorliegt.

Somit ist es möglich, die Reibung entlang einer Absaugstrecke zu reduzieren. Am distalen Ende des Absaugkanals 3 weist dieser einen engeren Querschnitt auf, so dass hier das abgetrennte Bioptat relativ großflächig an einer Absaugkanalinnenseite 12 anliegt. Nach Anlegen des Unterdrucks, insbesondere eines Vakuums, steigt eine Druckdifferenz zwischen herrschendem Außendruck und dem an der dem Schneidewerkzeug 7 abgewandten Seite des Bioptats anliegenden Drucks innerhalb des Absaugkanals 3 so weit an, bis das Bioptat davon mitgerissen wird. Die anfängliche Beschleunigung des Bioptats ist dabei ausreichend, um das Bioptat bis in den proximalen Bereich 5 zu befördern, wo es gegebenenfalls durch einen Gewebekollektor aufgefangen wird.

Durch den sich aufweitenden Absaugkanal 3 ist die Reibung und/oder der Kontakt zwischen dem Bioptat und der Wandung 17 des Absaugkanals 3 entlang der Absaugstrecke geringer als am distalen Bereich 4, also in einer Anfangsposition des Bioptats, nachdem dieses durch das Schneidewerkezug abgetrennt wurde.

Das chirurgische Instrument 1 kann je nach Bedarf unterschiedliche Schneidewerkzeuge 7 aufweisen.

In den Figuren 1-3 ist ein Ausführungsbeispiel gezeigt, das als Kerrison Stanze ausgebildet ist, wobei das Schneidewerkzeug 7 als Bajonett ausgebildet. Hierbei weist das Schneidewerkzeug 7 ein feststehendes Schneidteil 8 und relativ dazu entlang einer Längsachse axial verstellbares Gegenschneidteil 9 auf.

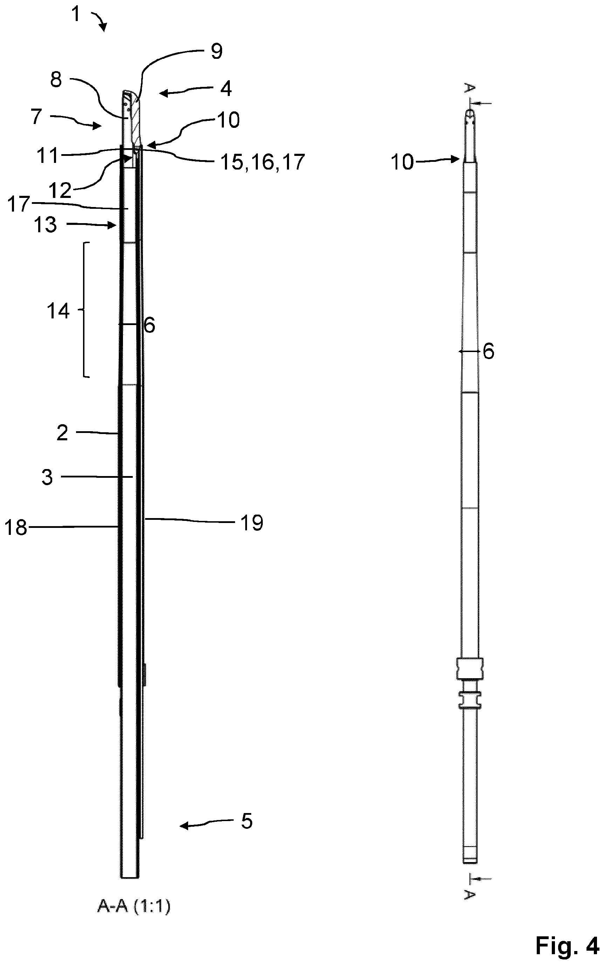

In den Figuren 4 bis 6 ist ein zweites Ausführungsbeispiel gezeigt, dessen Schneidewerkzeug 7 eine Maulgeometrie aufweist. Das Schneidewerkzeug 7 weist ein feststehendes Schneidteil 8 und ein um eine durch ein Drehgelenk 10 ausgebildete Drehachse relativ zum feststehenden Schneidteil 8 verstellbares Gegenschneidteil 9 auf. Bei derartigen chirurgischen Instrumenten 1 wird das zuvor genannte Drehgelenk 10 durch wenigstens ein Befestigungselement 11 ausgebildet. Wird nur ein, beispielsweise stabförmiges Befestigungselement 11 verwendet, das senkrecht zur Längsachse des Absaugkanals 3 von einer Seite des Schaftes 2 zur anderen Seite verläuft, so bildet das Befestigungselement 11 innerhalb des Absaugkanals 3 ein Hindernis aus. Dieses Hindernis kann zu einer Beschädigung des Bioptats beim Absaugen und/oder im schlimmsten Falle zu einer Verstopfung des Absaugkanals 3 durch Festsetzen des Bioptats am Befestigungselement 11 führen.

Selbst bei der Ausgestaltung des Drehgelenkes 10 durch zwei gegenüberliegende Befestigungselemente 11 ragen die Befestigungselemente 11 vorbekannter Instrumente ungünstig in den Absaugkanal 3 hinein, da diese aufgrund der vereinfachten Fertigung von außen nach innen eingesteckt sind. Daher liegen die Köpfe 16 der Befestigungselemente 11 vorbekannter Instrumente an der Außenseite 13 des Schaftes.

Bei der Ausgestaltung aus den Figuren 4-6 ist es vorgesehen, dass die Stirnseiten 15 der Befestigungselemente 11 in einer Absaugkanalsinnenseite 12 liegen. An den Stirnseite 15 sind in der Regel breitere Köpfe 16 ausgebildet, um ein Herausrutschen der Befestigungselemente 11 aus den Befestigungsöffnungen in der Wandung 17 des Absaugkanals 3 zu verhindern. Durch die Einführung der Befestigungselemente 11 von einer Innenseite der Wandung 17 nach außen ist es möglich, scharfkantige Strukturen an der Absaugkanalinnenseite 12 zu verhindern.

Die Querschnittserweiterung kann in unterschiedlicher Art und Weise ausgebildet sein. So kann diese zum Beispiel kontinuierlich und/oder geradlinig sein. Sie kann sich über den gesamten Absaugkanal 3 oder nur über einen Abschnitt des Absaugkanals 3 (Erweiterungsbereich 14) erstrecken.

Bei einer Ausgestaltung, bei welcher die Querschnittserweiterung in einem Erweiterungsbereich 14 auftritt, kann es vorgesehen sein, dass der Absaugkanal 3 im Abschnitt vor und/oder nach dem Erweiterungsbereich 14 einen gleichdimensionierten Querschnitt 6 und/oder in der Form gleichbleibenden Querschnitt 6 aufweist.

Der Erweiterungsbereich 14 kann sich, wie in den Figuren 1-6 gezeigt ist, vom distalen Ende des Schaftes 2 bis maximal zu einer Mitte des Schaftes 2, insbesondere bis maximal zu einem Drittel einer Länge des Schaftes 2, erstrecken.

Bei der Ausgestaltung aus den Figuren 4-6 bilden die Stirnseiten 15 der Befestigungselemente 11, vorzugsweise also die Stirnseiten 15 der Köpfe 16 der Befestigungselemente 11, zusammen mit der Wandung 17 des Absaugkanals 3 eine planare Absaugkanalsinnenseite 12. Ein Übergang zwischen einem Befestigungselement 11 und der Wandung 17 kann dabei glatt und/oder stufenlos ausgestaltet sein. Somit kann noch besser verhindert werden, dass eine Gewebeprobe an dem Befestigungselement 11 hängen bleibt und dadurch zerstört wird und/oder dass es im Bereich des Drehgelenks 10 innerhalb des Absaugkanals 3 zu einer Verstopfung kommt. Die Köpfe der Befestigungselemente 11 können wenigstens teilweise in eine Materialanpassung der Wandung 17 versenkt sein.

Um noch besser verhindern zu können, dass ein Bioptat am Befestigungselement 11 hängen bleibt, können die an der Absaugkanalinnenseite 12 liegenden Kanten der Befestigungselemente 11 wenigstens teilweise abgerundet sein. Alternativ oder ergänzend dazu kann die Absaugkanalinnenseite 12 teilweise ausbildenden Stirnflächen 15 konvex gewölbt sein.

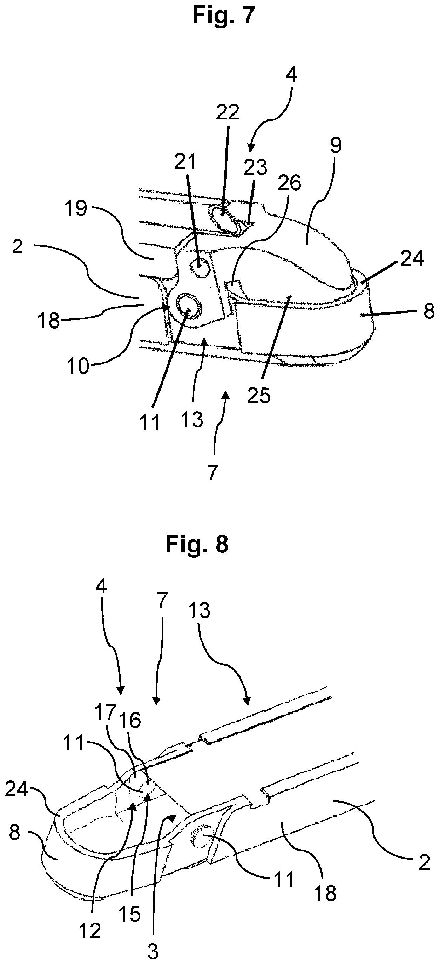

Fig. 7 und Fig. 8 zeigen einen alternativ ausgestalteten distalen Bereich 4 eines weiteren Ausführungsbeispiels eines erfindungsgemäß ausgebildeten chirurgischen Instruments 1. In Fig. 8 sind einige Teile ausgeblendet, um einen Blick von innen auf die Befestigungselemente 11 zu ermöglichen.

Das Schneidewerkzeug 7 weist ein feststehendes Schneidteil 8 und ein hiergegen verstellbares Gegenschneidteil 9 auf. Das Gegenschneidteil 9 ist an dem Drehgelenk 10 gegenüber dem Schneidteil 8 verdrehbar. Das Gegenschneidteil 9 ist über das Gelenk 21 mit der Gleitschiene 19 gelenkig verbunden. Die Gleitschiene 19 kann wie zuvor beschrieben auf der Führungsschiene 18 gleiten. Wird die Gleitschiene 19 nach vorne geschoben, so dreht sich das Gegenschneidteil 9 um das Drehgelenk 10 in eine Öffnung des Schneidteils 8. Das Gegenschneidteil 9 hat lediglich ein geringes Spiel innerhalb eines U-förmig ausgebildeten Rands 24 des Schneidteils 8, sodass Gewebe, das zwischen das Gegenschneidteil 9 und das Schneidteil 8 gelangt, ausgestanzt oder abgeschnitten wird. Hierzu weist das Gegenschneidteil 9 eine Schneide 25 auf.

An der Oberseite der Gleitschiene 19 ist eine Zuleitung 22 ausgebildet, durch die insbesondere eine Flüssigkeit zuführbar ist. Ist die Gleitschiene 19 zurückgeschoben, so greift eine Spitze der Zuleitung 22 in eine Aussparung 23 des Gegenschneidteils 9 ein, sodass die Flüssigkeit direkt in einen zwischen dem Gegenschneidteil 9 und dem Schneidteil 8 ausgebildeten Stanzraum eingeleitet werden kann.

Wie aus Fig. 8 genauer ersichtlich, ist das Drehgelenk 10 dadurch gebildet, dass beidseitig des Schafts 2 jeweils ein Befestigungselement 11 ausgebildet ist, welches das an dieser Stelle innen gelegene feststehende Schneidteil 8 und das an dieser Stelle außen gelegene verstellbare Gegenschneidteil 9 miteinander verdrehbar verbindet. In dem Gegenschneidteil 9 ist eine Ausnehmung 26 ausgebildet, sodass ein vorderer Bereich des Gegenschneidteils 9 nicht mehr außen gelegen ist, sondern in das Schneidteil 8 innenseitig eingreifen kann.

Wie aus Fig. 8 ersichtlich, sind die Befestigungselemente 11 von innen nach außen durch eine Bohrung einer Wandung 17 des Absaugkanals 3 eingebracht. Hierbei ist die Bohrung innenseitig breiter ausgebildet als außenseitig, sodass der Kopf 16 des Befestigungselements 11, der breiter als der restliche Bereich des Befestigungselements 11 ausgebildet ist, vollständig in der Wandung 17 versenkbar ist. Hierbei ist eine Innenfläche der Bohrung auf eine Geometrie des Befestigungselements 11 abgestimmt.

Es ist in Fig. 8 erkennbar, dass die Stirnflächen 15 der Befestigungselemente 11 zusammen mit der Wandung 17 des Absaugkanals 3 eine planare Absaugkanalsinnenseite 12 bilden. Zugleich ist ein Übergang zwischen dem Kopf 16 und der Wandung 17 glatt und stufenlos ausgestaltet.

Bei dem in Fig. 7 und Fig. 8 gezeigten Ausführungsbeispiel kann wie zuvor beschrieben ein Durchmesser des Querschnitts 6 des Absaugkanals 3 in Absaugrichtung vom distalen Bereich 4 zum proximalen Bereich 5 erweitert sein.

### Bezugszeichenliste

- 1: Chirurgisches Instrument
- 2: Schaft
- 3: Absaugkanal
- 4: Distaler Bereich
- 5: Proximaler Bereich
- 6: Querschnitt
- 7: Schneidewerkzeug
- 8: Feststehendes Schneidteil
- 9: Verstellbares Gegenschneidteil
- 10: Drehgelenk
- 11: Befestigungselement
- 12: Absaugkanalinnenseite
- 13: Außenseite des Schaftes
- 14: Erweiterungsbereich
- 15: Stirnseite; Stirnfläche
- 16: Kopf des Befestigungselements
- 17: Wandung des Absaugkanals
- 18: Führungsschiene
- 19: Gleitschiene
- 20: Griffteil
- 21: Gelenk
- 22: Zuleitung
- 23: Aussparung
- 24: Rand
- 25: Schneide
- 26: Ausnehmung

## Patentansprüche

1. **Chirurgisches Instrument (1)** zum Abtrennen und Entfernen eines Bioptats,
- wobei in einem Schaft (2) des Instruments (1) ein Absaugkanal (3) zum Absaugen des Bioptats aus einem distalen Bereich (4) in Richtung eines proximalen Bereichs (5) ausgebildet ist und
- wobei in dem distalen Bereich (4) des Schaftes (2) ein Schneidewerkzeug (7) ausgebildet ist, das ein Schneidteil (8) und ein bewegliches Gegenschneidteil (9) aufweist,
- wobei das Gegenschneidteil (9) um eine Drehachse, die durch ein Drehgelenk (10) ausgebildet ist, relativ zum Schneidteil (8) verstellbar ist, wobei das Drehgelenk (10) durch zwei Befestigungselemente (11) ausgebildet ist, die jeweils von innen nach außen eingeführt sind, wobei sich innen auf eine Absaugkanalsinnenseite (12) des Absaugkanals (3) bezieht
**dadurch gekennzeichnet dass** das Schneidteil (8) feststehend ist und
- dass die Stirnseiten (15) der Befestigungselemente (11) zusammen mit einer Wandung (17) des Absaugkanals (3) eine planare Absaugkanalsinnenseite (12) bilden.

2. Chirurgisches Instrument (1) nach Anspruch 1, wobei sich der Querschnitt (6) des Absaugkanals (3) in einem Erweiterungsbereich (14) erweitert, insbesondere kontinuierlich und/oder geradlinig erweitert.

3. Chirurgisches Instrument (1) nach Anspruch 2, wobei der Absaugkanal (3) vor und/oder nach dem Erweiterungsbereich (14) einen gleichdimensionierten Querschnitt (6) und/oder einen in der Form gleichbleibenden Querschnitt (6) aufweist.

4. Chirurgisches Instrument (1) nach Anspruch 2 oder 3, wobei sich der Erweiterungsbereich (14) von einem distalen Ende des Schaftes (2) bis maximal zu einer Mitte des Schaftes (2), insbesondere bis maximal zu einem Drittel einer Länge des Schaftes (2), erstreckt.

5. Chirurgisches Instrument (1) nach einem der vorstehenden Ansprüche, wobei die Stirnseiten (15) der Befestigungselemente (11), insbesondere Köpfe (16) der Befestigungselemente (11), in der Absaugkanalsinnenseite (12) liegen.

6. Chirurgisches Instrument (1) nach Anspruch 5, wobei die Stirnseiten (15) der Köpfe (16) der Befestigungselemente (11) zusammen mit der Wandung (17) des Absaugkanals (3) die planare Absaugkanalsinneneite 12) bilden,
- insbesondere wobei ein Übergang zwischen einem der Köpfe (16) und der Wandung (17) glatt und/oder stufenlos ausgestaltet ist.

7. Chirurgisches Instrument (1) nach Anspruch 5 oder 6, wobei Kanten der Köpfe (16) der Befestigungselemente (11) wenigstens teilweise abgerundet und/oder die Stirnflächen (15) konvex gewölbt sind.

8. Chirurgisches Instrument (1) nach einem der vorstehenden Ansprüche, wobei der Schaft (2) eine Führungsschiene (18) und eine relativ zur Führungsschiene (18) verstellbare Gleitschiene (19) aufweist und/oder
- wobei der Absaugkanal (3) teilweise oder vollständig durch eine Führungsschiene (18) und/oder eine Gleitschiene (19) ausgebildet ist.

9. **Verfahren zur Herstellung eines chirurgischen Instruments** (1) nach einem der vorstehenden Ansprüche 1 bis 8,
**dadurch gekennzeichnet,**
- **dass** die zur Ausbildung des Drehgelenks (10) verwendeten Befestigungselemente (11) durch die Wandung (17) des Absaugkanals (3) von innen nach außen in Öffnungen eingeführt und darin fixiert werden und zwar derart,
- **dass** die Stirnseiten (15) der Befestigungselemente (11) und eine Innenseite der Wandung (17) die planare Absaugkanalsinnenseite (12) des Absaugkanals (3) ausbilden.

10. **Verwendung eines Drehgelenks (10)**, welches zwei von innen eingesteckte Befestigungselemente (11) aufweist, zur Ausbildung eines Schneidewerkzeugs (7), welches in einem distalen Bereich (4) eines Schaftes (2) eines chirurgischen Instruments (1) gemäß einem der vorstehenden Ansprüche 1 bis 8 ausgebildet ist, **dadurch gekennzeichnet,**
- **dass** die Befestigungselemente (11) dazu verwendet werden, die Absaugkanalsinnenseite (12) des Absaugkanals (3) planar auszugestalten.

## Claims

1. **Surgical instrument (1)** for severing and removing a biopsy specimen,
- wherein a suction channel (3) for aspiration of the biopsy specimen from a distal region (4) in the direction of a proximal region (5) is formed in a shaft (2) of the instrument (1), and
- wherein a cutting tool (7) is formed in the distal region (4) of the shaft (2), the cutting tool having a cutting part (8) and a movable counter cutting part (9),
- wherein the counter cutting part (9) is adjustable relative to the cutting part (8) about an axis of rotation which is formed by a rotary joint (10), wherein the rotary joint (10) is formed by two fastening elements (11) which are each introduced from the inside outwards, wherein on the inside refers to a suction channel inner side (12) of the suction channel (3),
**characterized in that** the cutting part (8) is fixed, and
- **in that** the end sides (15) of the fastening elements (11) together with a wall (17) of the suction channel (3) form a planar suction channel inner side (12).

2. Surgical instrument (1) according to Claim 1, wherein the cross section (6) of the suction channel (3) widens in a widening region (14), in particular widens continuously and/or rectilinearly.

3. Surgical instrument (1) according to Claim 2, wherein the suction channel (3) has a uniformly dimensioned cross section (6) and/or a cross section (6) which is consistent in shape upstream and/or downstream of the widening region (14).

4. Surgical instrument (1) according to Claim 2 or 3, wherein the widening region (14) extends from a distal end of the shaft (2) as far as at maximum a centre of the shaft (2), in particular as far as at maximum a third of a length of the shaft (2).

5. Surgical instrument (1) according to one of the preceding claims, wherein the end sides (15) of the fastening elements (11), in particular heads (16) of the fastening elements (11), lie in the suction channel inner side (12).

6. Surgical instrument (1) according to Claim 5, wherein the end sides (15) of the heads (16) of the fastening elements (11) together with the wall (17) of the suction channel (3) form the planar suction channel inner side (12),
- in particular wherein a transition between one of the heads (16) and the wall (17) is configured to be smooth and/or continuous.

7. Surgical instrument (1) according to Claim 5 or 6, wherein edges of the heads (16) of the fastening elements (11) are at least partially rounded and/or the end surfaces (15) are curved convexly.

8. Surgical instrument (1) according to one of the preceding claims, wherein the shaft (2) has a guide rail (18) and a sliding rail (19) which is adjustable relative to the guide rail (18), and/or
- wherein the suction channel (3) is formed partially or completely by a guide rail (18) and/or a sliding rail (19).

9. **Method for producing a surgical instrument** (1) according to one of the preceding Claims 1 to 8,
**characterized**
- **in that** the fastening elements (11) used for forming the rotary joint (10) are introduced from the inside outwards through the wall (17) of the suction channel (3) into openings and fixed therein, specifically in such a manner
- that the end sides (15) of the fastening elements (11) and an inner side of the wall (17) form the planar suction channel inner side (12) of the suction channel (3).

10. **Use of a rotary joint (10)**, which has two fastening elements (11) inserted from the inside, for forming a cutting tool (7) which is formed in a distal region (4) of a shaft (2) of a surgical instrument (1) according to one of the preceding Claims 1 to 8, **characterized**
- **in that** the fastening elements (11) are used to configure the suction channel inner side (12) of the suction channel (3) to be planar.

## Revendications

1. **Instrument chirurgical (1)** pour séparer et enlever un échantillon de biopsie,
- un canal d'aspiration (3) pour aspirer l'échantillon de biopsie d'une zone distale (4) en direction d'une zone proximale (5) étant formé dans une tige (2) de cet instrument (1) et
- un outil de coupe (7) qui comporte une pièce de coupe (8) et une pièce de coupe opposée mobile (9) étant formé dans la zone distale (4) de la tige (2),
- laquelle pièce de coupe opposée (9) peut être réglée par rapport à la pièce de coupe (8) autour d'un axe de rotation qui est formé par une articulation rotoïde (10),
- laquelle articulation rotoïde (10) est formée par deux éléments de fixation (11) qui sont introduits chacun de l'intérieur vers l'extérieur, l'intérieur se rapportant à la face interne de canal d'aspiration (12),
**caractérisé en ce que** la pièce de coupe (8) est fixe et que
- les faces frontales (15) des éléments de fixation (11) forment avec une paroi (17) du canal d'aspiration (3) une face interne de canal d'aspiration (12) plane.

2. Instrument chirurgical (1) selon la revendication 1, dans lequel la section transversale (6) du canal d'aspiration (3) s'élargit dans une zone d'élargissement (14), en particulier s'élargit de façon continue et/ou rectiligne.

3. Instrument chirurgical (1) selon la revendication 2, dans lequel le canal d'aspiration (3) présente, devant ou derrière la zone d'élargissement (14), une section transversale (6) de dimension constante et/ou une section transversale (6) de forme constante.

4. Instrument chirurgical (1) selon la revendication 2 ou 3, dans lequel la zone d'élargissement (14) s'étend d'une extrémité distale de la tige (2) jusqu'à un milieu au maximum de la tige (2), en particulier jusqu'à un tiers au maximum d'une longueur de la tige (2).

5. Instrument chirurgical (1) selon une des revendications précédentes, dans lequel les faces frontales (15) des éléments de fixation (11), en particulier des têtes (16) des éléments de fixation (11), se trouvent dans une face interne de canal d'aspiration (12).

6. Instrument chirurgical (1) selon la revendication 5, dans
lequel les faces frontales (15) des têtes (16) des éléments de fixation (11) forment avec la paroi (17) du canal d'aspiration (3) la face interne de canal d'aspiration (12) plane,
- en particulier un passage entre une des têtes (16) et la paroi (17) étant lisse et/ou continu.

7. Instrument chirurgical (1) selon la revendication 5 ou 6, dans lequel des bords des têtes (16) des éléments de fixation (11) sont au moins partiellement arrondis et/ou les faces frontales (15) sont courbées de manière convexe.

8. Instrument chirurgical (1) selon une des revendications précédentes, dans lequel la tige (2) comporte un rail de guidage (18) et une glissière (19) réglable par rapport au rail de guidage (18) et/ou
- dans lequel le canal d'aspiration (3) est partiellement ou entièrement formé par un rail de guidage (18) et/ou une glissière (19).

9. **Procédé de production d'un instrument chirurgical** (1) selon une des revendications précédentes 1 à 8, **caractérisé en ce que**
- les éléments de fixation (11) utilisés pour former l'articulation rotoïde (10) sont introduits à travers la paroi (17) du canal d'aspiration (3) de l'intérieur vers l'extérieur dans des orifices et fixés dans ceux-ci, de telle sorte que
- les faces frontales (15) des éléments de fixation (11) et une face interne de la paroi (17) forment la face interne de canal d'aspiration (12) plane du canal d'aspiration (3).

10. **Utilisation d'une articulation rotoïde (10)** qui présente deux éléments de fixation (11) insérés de l'intérieur pour former un outil de coupe (7), lequel est formé dans une zone distale (4) d'une tige (2) d'un instrument chirurgical (1) selon une des revendications précédentes 1 à 8, **caractérisée en ce que**
- les éléments de fixation (11) sont utilisés pour former de manière plane la face interne de canal d'aspiration (12) du canal d'aspiration (3).
